# EUROPEAN PATENT APPLICATION

(11) **EP 4 066 770 A1**
(43) Date of publication of application: **05.10.2022**
(21) Application number: 21305433.1
(22) Date of filing: 02.04.2021
(51) Int. Cl.: A61B 34/00, A61B 17/00

(54) **DELIVERY AND GATHERING SYSTEM COMPRISING A MICROROBOT**

(71) Applicant: Robeaute, 75002 Paris (FR)
(72) Inventor: DUPLAT, Bertrand, 75002 Paris (FR); OULMAS, Ali, 75012 Paris (FR); FRANCOIS, Quentin, 75116 Paris (FR)
(74) Representative: Icosa

(57) **Abstract**

Delivery and gathering system (10) for at least one target point inside a target body part of a patient, the system (10) comprising:
- a microrobot (12) aimed at navigating towards the at least one target point,
- a cap aimed at being secured to a securing body part of the patient, the cap (14) comprising a channel which enables to introduce elements into the target body part, or enables to remove elements from inside the target body part,
- a wire (16) connected to the microrobot (12) and to the cap (14),
- a control unit (18) configured to communicate with the cap (14), the control unit (18) comprising a user interface enabling a user to remotely control and navigate the microrobot (12),
wherein the system (10) further comprise a flexible elongated element (20) configured to cooperate, by sliding, with the wire (16) in order to extend, from the cap (14), towards the target point.

## Description

### FIELD OF INVENTION

The present invention relates to remote medical or physiological actions in a patient's body, for example remote drug delivery and or body fluid gathering.

### BACKGROUND OF INVENTION

Medical microrobots and nanorobot, including wired microrobots/nanorobot, are becoming a fundamental diagnostic and therapeutic tool of medicine regarding situations in which it is necessary to get to hard too reach locations through difficult micro-intrusive routes.

However, the autonomy of those microrobots or nanorobots is low and does not enable the robot to travel far enough or long enough in order to reach specific hard to reach locations located deep inside the brain and not at the periphery thereof.

Once at the target location (or locations), the microrobots might be able to perform various measures by means of embedded sensors and/or perform several tasks by means of effectors. The microrobot may also have to gather some biological material either liquid or solid and bring it back out of the body for study, and/or locally release materials in the body (cells, drugs or other molecules) as part of a therapy.

However, such a microrobot may be unable to carry enough material either gathered from the location or in order to deliver it to said location. More particularly, because of their relatively small size, in some cases due to limitations of diagnosis technologies, the microrobots might not be able to gather enough material for an efficient biopsy. This is also true for drugs deliveries, when larger quantities that they can carry aboard might be needed.

The aim of this invention is to provide a safe and reliable system for reaching difficult to reach target points inside target body parts, in order to act upon those target points.

### SUMMARY

This invention thus relates to a delivery and gathering system aimed at delivering or gathering material, energy, information or structural elements from or to at least one target point inside a target body part of a patient, the system comprising:
- a microrobot configured to navigate inside the target body part and aimed at navigating towards the at least one target point,
- a cap aimed at being secured to either a securing element secured to a securing body part of the patient, or directly to the securing body part, the cap comprising a channel which enables to introduce elements or element parts into the target body part, or enables to remove elements or element parts from inside the target body part,
- a wire connected to the microrobot on one end and to the cap on the other end,
- a control unit configured to communicate with the cap, the control unit comprising a user interface enabling a user to remotely control and navigate the microrobot,
wherein the system further comprise a flexible elongated element configured to cooperate, by sliding, with the wire in order to extend, from the cap, through the target body part, towards the target point, the wire being thus aimed at guiding the flexible elongated element towards the target point.

This way, the solution enables to get or gather more material in a safe and quick way to target points located in difficult to reach areas. This may also enable to deliver larger elements or larger quantities of fluid to very precise locations.

The system according to the invention may comprises one or several of the following features, taken separately from each other or combined with each other:
- the flexible elongated element displays a general tubular shape, the flexible elongated element being configured to slide around the wire,
- the flexible elongated element is a catheter configured to cooperate with the wire and the cap in order:
   - to deliver material from the cap towards the at least one target point, or
   - to gather material from the at least one target point towards the cap,
- the cap comprises a pump configured to pump fluids and material through the flexible elongated element,
- the flexible elongated element is an electrode or a sensor configured to gather information from the at least target point or to deliver energy to said at least target point,
- the wire provides energy to the microrobot,
- the cap comprises a wire winding system in order to control the wire extension inside the target body part, the wire winding system being motorized and controlled by the control unit, the wire winding system further comprising at least one monitoring sensor,
- the cap comprises a flexible elongated element winding system in order to control the extension of the flexible elongated tubular element inside the target body part, the flexible elongated element winding system being motorized and controlled by the control unit, the flexible elongated element winding system further comprising at least one monitoring sensor,
- the flexible elongated element comprises at least one monitoring sensor at its distal end,
- the flexible elongated element comprises an adaptative diameter on at least one deformable portion,
- the flexible elongated element comprises a garage alike structure in order to store the micro-robot,
wherein the flexible elongated element comprises at least one tracker enabling to monitor, in real time, the extension of the flexible elongated element.

The present invention also relates to a material delivery and gathering method implemented by means of the system according to any one of the preceding features, the method comprising following steps in the order of enunciation:
- securing the cap to the securing body part of the patient,
- programming a microrobot navigation route by means of the user interface of the control unit,
- securing, if necessary, one end of the wire to the cap,
- securing, if necessary, the other end of the wire to the microrobot,
- inserting the microrobot inside the target body part of the patient through the channel of the cap,
- inserting the distal end of the flexible elongated element inside the channel of the cap,
- setting the flexible elongated element and the wire up for cooperation,
- setting the microrobot into motion towards the at least one target point,
- monitoring the wire extension through the target body part,
- monitoring the flexible elongated element extension through the target body part,
- once the microrobot has reached the at least one target point, activating the material, energy, information or structural elements material delivery or gathering, through the flexible elongated element,
- once the delivery or gathering has been completed, either proceed to delivery or gathering to a further target point, or remove the flexible elongated element, the wire and the microrobot from the target body part, through the cap.

The method may further include following steps:
- microrobot is removed by being pulled through the flexible elongated element,
- the microrobot is stored in a garage alike structure of the flexible elongated element prior to be removed from the target body part.

### BRIEF DESCRIPTION OF THE DRAWINGS

The invention will be better understood, and other aims, details, characteristics and advantages thereof will emerge more clearly on reading the detailed description which follows, of one or several embodiments of the invention given by way of illustration. Those are purely illustrative and non-limiting examples, with reference to the accompanying schematic drawings.

On these drawings:
- **Figure 1** is a schematic general view of a first embodiment of the system according to the present invention,
- **Figure 2** is a schematic general view of a second embodiment of the system according to the present invention,
- **Figure 3** is a schematic general view of a third embodiment of the system according to the present invention,
- **Figure 4A** is a schematic view of the elongated element - wire cooperation regarding an alternative embodiment of the current invention,
- **Figure 4B** is a schematic view of the elongated element - wire cooperation regarding another alternative embodiment of the current invention,
- **Figures 5A to 5C** are perspective schematic views of different embodiments of the flexible elongated element according to the present invention,
- **Figure 6** is a schematic depiction of the method according to the current invention according a first embodiment,
- **Figure 7** is a schematic depiction of the method according to the current invention according a second embodiment.

### DETAILED DESCRIPTION

The delivery and gathering system 10 according to the present invention aims at gathering material, energy, information or structural elements from or to at least one target point inside a target body part of a patient.

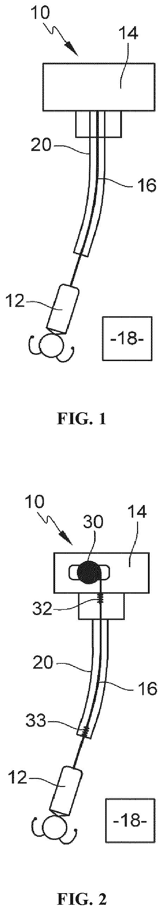

As can be seen on figures 1, 2 and 3, the system 10 according to the present invention comprises:
- a microrobot 12 configured to navigate inside the target body part and aimed at navigating towards the at least one target point,
- a cap 14 aimed at being secured to a securing body part of the patient,
- a wire 16 connected to the microrobot 12 on one end and to the cap 14 on the other end,
- a control unit 18 configured to communicate with the cap 14,
- a flexible elongated element 20 configured to cooperate, by sliding, with the wire 16, in order to extend, from the cap 14, through the target body part, towards the target point.

The microrobot 12 is either self-propelled with an internal engine or propelled with external means such as coils moving magnet(s) embedded inside the microrobot 12. The microrobot 12 can be 0.1 to 5mm, preferably 2mm in diameter, and up to 5cm, preferably 2cm, in length.

The wire 16 is secured on one end to the cap 14 and, on the other end, to the microrobot 12. It can actually be made out of multiple wires. It may, regarding certain embodiments, provide energy (fluidic, electrical or optical) to the microrobot 12. The wire 16 further allows communication (fluidic, electrical or optical) to and from the microrobot 12. The wire 12 further provides a strong hold on the microrobot 12, allowing the microrobot 12 to be pulled back out of the patient's body in case of an emergency.

The flexible elongated element 20 is configured to be guided by the wire 16 towards the target point.

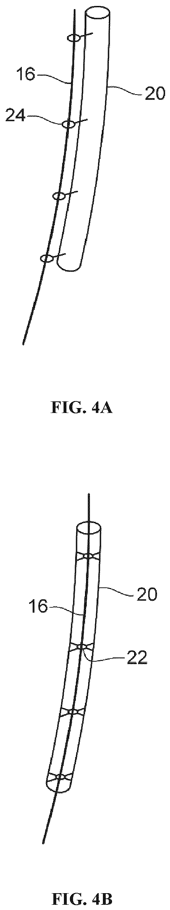

In some embodiments, the flexible elongated element 20 displays a general tubular shape and it slides around the wire 16. In this instance of the invention, the flexible elongated element 20 is centered around the wire 16 and is naturally guided by it. In other embodiments, centering structures 22 within the flexible elongated element 20 can also help guide and center the flexible elongated element 20 around the wire 16 (see FIG. 4B) In another embodiment shown on FIG. 4A, the flexible elongated element 20 comprises cooperating means 24 on its side, said cooperating means 24 cooperating with the wire 16 in order to hold and follows it.

In some embodiments, the flexible elongated element 20 comprises several concentric layers, forming thus at least two concentric embedded tubular elements. Those tubular elements might be isolated from each other or in fluidic or energetic communication with each other. This way, a first device or fluid might circulate in the external tubular element while a second device or fluid might circulate in the internal tubular element.

In some other embodiments, the flexible elongated element 20 may comprise several structural zones along its length, each structural zone displaying different structural features, for example different wall colors or opacity, or walls made of different materials, for example.

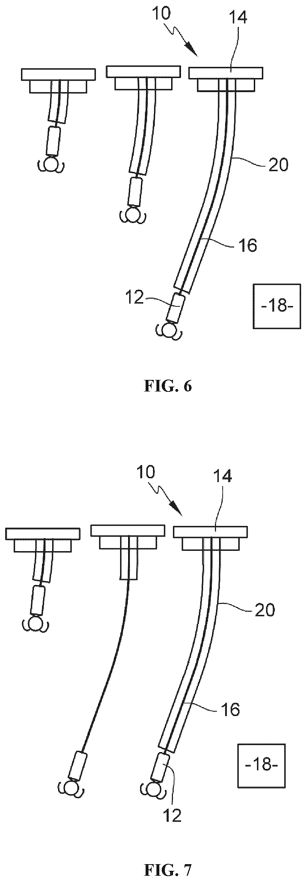

The way the flexible elongated element 20 is moving forward (meaning extending through the target body part) relatively to the wire 16 can follow different rules, like illustrated on FIG. 6 and FIG. 7:
- the flexible elongated element 20 can be moved at the same time and rhythm as the microrobot 12 at a slight distance behind it. This distance can be fully bridged, if necessary, once the microrobot 12 has reached the target point (see FIG. 6),
- the flexible elongated element 20 can be extended only once the microrobot 12 has reached the at least one target point. Then it is fully extended until it touches or gets close from the microrobot 12. (see FIG. 7),
- the flexible elongated element 20 can further be extended in successive steps, for instance when the microrobot 12 is about to make a significant rotary shift in direction (inside a natural bodily fluid channel or progressing in tissues, for example), or once a certain distance has been covered by the microrobot 12 inside the target body part,

Other rules for the flexible elongated element 20 motions can be of course envisioned.

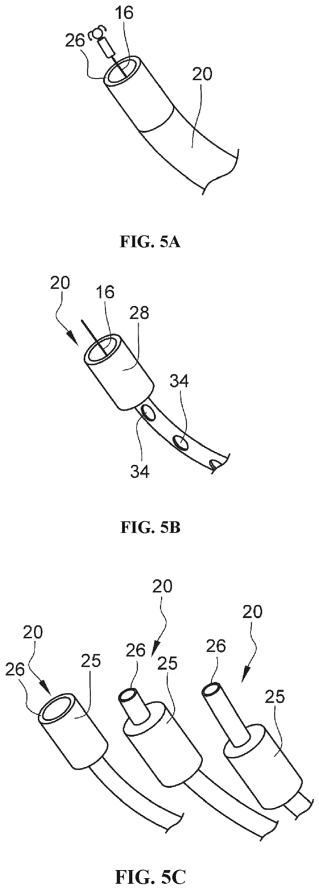

Considering the embodiments in which the flexible elongated element 20 displays a general tubular shape, the flexible elongated element 20 can have a definite fixed diameter (see FIG. 5A) or can have a variable adaptable diameter (see FIG. 5C). The adaptative diameter can be adaptative through the whole length of the flexible elongated element 20 (keeping the same diameter through the whole flexible elongated element 20), or adaptive on a deformable portion 25 of the length of the flexible elongated element 20 and potentially a moving deformable portion 25, like illustrated on FIG. 5C. This moving of the deformable portion 25 is generated by a moving element or material inserted inside the flexible elongated element 20, said element or material moving upwardly or downwardly inside the flexible elongated element 20. In some cases, said element can be the microrobot 12 itself, after the target point has been reached and the microrobot 12 has to be safely removed from the target body part of the patient. The deformation of the flexible elongated element 20 can be achieved through extensible materials such as those found on stents, or « origami-like » folding that can unfold through pressure (fluid pressure or microrobot pressure).

The flexible elongated element 20 has two ends: one free end, the distal end 26, and one proximal end (not shown) which might be secured to the cap 14 or elsewhere.

In some, not represented, embodiment, the flexible elongated element 20 may carry a camera or any other recording device, or a fiberglass at its distal end 26. In some further not represented embodiments, the flexible elongated element 20 may carry several cameras or any other recording device along its length.

The relationship between the microrobot 12 and the flexible elongated element 20 can take different forms depending on various embodiments of the invention:
- the flexible elongated element 20 can have its distal end 26 either always behind or in simple contact with the microrobot 12 once each target point has been reached,
- the flexible elongated element 20 can be slightly wider that the microrobot 12 so that, if the flexible elongated element 20 displays a general tubular shape, the microrobot 12 can be inserted back in the flexible elongated element 20 and potentially even extracted back through the flexible elongated element 20 fully to the cap 14 as already mentioned above,
- the flexible elongated (tubular) element 20 can have the same width than the microrobot 12 or even a smaller one but be endowed with local stretching capabilities so as to « swallow » back the microrobot 12 and let it glide through to potentially extract it out in a peristaltic way, as snakes do when they ingest a prey wider than them,
- the flexible elongated (tubular) element 20 can comprise a garage alike structure 28 (see FIG. 5B) at its distal end 26 (or anywhere else on its length) that is either larger than the microrobot 12, or extensible so to « swallow » the microrobot 12 and for long periods as in a garage. The garage alike structure 28 could also have the ability to fully close and be hermetic. When the flexible elongated (tubular) element 20 is extracted from the target body part, then the microrobot 12 in the garage alike structure 28 is extracted together with it.

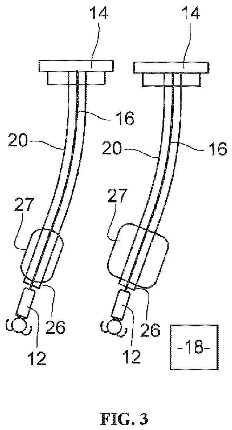

The distal end 26 of the can be sometimes left loose but other times it might be necessary to block it. It might be necessary to block it at its distal extremity 26 or in several points along its length. When the flexible elongated element 20 lies within a bodily channel (such as veins, arteries, bile or pancreatic duct, etc.) its distal end 26 can either:
- deploy a stent-like structure 27 that opens-up and so to block itself of the channel wall the same structure can get back in position later to unwind the flexible elongated element 20 back. (see FIG. 3),
- be attached with momentary adhesives (biological, chemical, charged static or using surface forces) of the natural bodily channel wall,
- use suction to attach on bodily channels walls or tissues through dedicated channels embedded in the flexible elongated element 20. The flexible elongated element 20 can comprise a tracker, for example, it can have patterns on its surface, including 3D patterns, that make it trackable, in real time, in 3D through Ultrasound, Scanner, MRI or even visually.

In some embodiments, the flexible elongated element 20 can be a catheter configured to cooperate with the wire 16 and the cap 14 in order:
- to deliver material from the cap 14 towards the at least one target point, and/or
- to gather material from the at least one target point towards the cap 14.

It is beneficial to use the wired microrobot 12 in conjunction with a flexible elongated element 20 as it allows to get more material in and out of the target body part than the microrobot 12 alone could.

In this case, the flexible elongated element 20 may allow to deliver, to the at least one target point:
- physical materials like drugs, cells, trophic factors, or
- structural elements like, for example, a stent.

In this case, the flexible elongated element 20 may further allow to gather (or remove), from the at least one target point:
- local fluids and tissues from the body, for example for biopsies, or even potentially during tumoral resections,
- structural elements.

More particularly, a flexible elongated element 20 can enable the system 10 to bring nanoparticles or any other particle having a local effect, like magnetic particles or air bubble (used for ultrasound localisation or imagery) or any other element that could be transported in a fluid.

In some alternative embodiment (not shown), the flexible elongated element 20 can be, for example, an electrode or a sensor configured to gather information from the at least target point or to deliver energy to said at least target point.

The cap 14 can be seen as a connector that connects the wire 16 and the flexible elongated element 20 with the external world meaning outside the patient's body. For doing so, the cap comprises a channel 22 which enables to introduce elements or element parts into the target body part, or enables to remove elements or element parts from inside the target body part. The cap is secured to the securing body part either by means of a securing element itself secured to the securing body part of the patient, like for example a trocar or a large catheter, or the cap 14 can be directly secured to the securing body part. Such a securing body part can be, for example, the skull, between vertebrae of the spine, or at the end/entrance of a natural body channel.

As can be seen on FIG. 2, the cap 14 may hold respective winding mechanism 30 of both wire 16 and flexible elongated element 20. Depending on the embodiments, the wire 16 is thus piloted by a motorized winder, located in the cap 14, dynamically managing the wire length as well as the wire tension. The cap 14 thus holds a motorized winding/unwinding mechanism 30 with, in some embodiments, at least one monitoring sensor 32 in order to manage the length and the tension of the wire 16. This monitoring sensor 32 might be a force sensor or any other sensor able to monitor force, speed and/or position. The angle of penetration of the wire 16 can also be managed. Such a winding mechanism 30 can help pushing the wire 16 so that it feels almost frictionless and weightless to the microrobot 12 and does not slow it down significantly or even stop it as it progresses in fluid or tissues of the target body part. In addition to the winding mechanism 30, or as an alternative to it, the flexible elongated element 20 may display, along the inside of its wall (or walls), a specific pattern in order to avoid any friction of the wire 16 along said wall(s). Said wall(s) may also be made, at least partially, of a material that has the same anti-frictional effect. In another embodiment, a small layer of fluid might stick on the inside of the wall(s) of the elongated flexible element 20. The flexible elongated element 20 is also piloted by a motorized winder mechanism (not shown), located in the cap 14, dynamically managing its length as well as its tension. The cap 14 thus holds a motorized winding/unwinding mechanism 30 with, in some embodiments, at least one monitoring sensor 32, managing length and tension of the flexible elongated element 20. The angle of penetration of the flexible elongated element 20 could also be managed. In an embodiment of the invention, the tension of the wire 16 is not only measured at its proximal extremity (in this case, the cap extremity) but also at the end of the flexible elongated element 20 with a dedicated monitoring sensor 33 (see FIG. 2). This is especially useful in scenarios where the flexible elongated element 20 does not wait for the microrobot 12 to have fully reached the target point before it unwinds. This makes sure that the tension on the last segment of the wire 16 (a few mm or cm long) is within the optimal functioning range so to make sure the microrobot 12 is not stuck or impeded in its course. Double checking the tension on both ends of the wire 16 (or the flexible element 20) lowers the risk of not detecting nonlinearities in the wire tension due to contact with the bodily environment and also potentially with the fluid inside the flexible elongated element 20 and its wall.

In case the flexible elongated element 20 displays general tubular shape (being, for example, a catheter), the cap 14 may also comprise in and out pumps to get fluids moving in and out through the flexible elongated element 20. The pumps (not represented) are located in the cap 14 in order to pump fluid in the body through the flexible elongated element 20 (potentially fluid charged with drugs) as well as potentially pump fluid out of the body, for instance in the case of liquid biopsies or micro-biopsies. Small solids such as individual cells or clusters of cells can be pressured to move in the flowing liquid.

The flexible elongated element 20 may comprise holes 34 in order to ease fluid gathering or delivery. Said holes 34 can be located at its tip or at various places through the flexible elongated element 20 length (see FIG. 5B).

The cap 14 further communicates with the control unit 18 and thus enables specially to connect the microrobot 12 and the wire 14 with the control unit 18.

The control unit 18 comprises a user interface enabling a user to remotely control and navigate the microrobot 12. It is by means of this user interface that any user can:
- define the routes and tasks of the microrobot 12, as well as
- follow the progress of the microrobot 12 in real time,
- monitor the extension of the wire 16 through the target body part,
- monitor the extension of the flexible elongated element 20 through the target body part,
- activate any gathering or delivering action once the microrobot 12 has reached the at least one target point.

The control unit 18 enables an operator to use the system 10 in order to implement a material delivery and gathering method, the method comprising following steps in the order of enunciation:
- securing the cap 14 to the securing body part of the patient, either directly or by means of a securing element,
- programming the microrobot navigation route by means of the user interface of the control unit 18,
- securing, if necessary, one proximal end of the wire 16 to the cap 14, in case, for example, the wire is a consumable material
- securing, if necessary, the distal end 26 of the wire 16 to the microrobot 12,
- inserting the microrobot 12 inside the target body part of the patient through the channel 22 of the cap 14,
- inserting the distal end 26 of the flexible elongated element 20 inside the channel 22 of the cap 14,
- setting the flexible elongated element 20 and the wire 16 up for cooperation by sliding,
- setting the microrobot 12 into motion towards the at least one target point,
- monitoring the wire 16 extension through the target body part,
- monitoring the flexible elongated element 20 extension through the target body part,
- once the microrobot 12 has reached the at least one target point, activating the material, energy, information or structural material delivery or gathering, through the flexible elongated element 20,
- once the delivery or gathering has been completed, either proceed to delivery or gathering to a further target point, or remove the flexible elongated element 20, the wire 16 and the microrobot 12 from the target body part, through the cap 14.

In some cases, the microrobot 12 is removed by being pulled through the flexible elongated element 20, and in some cases, the microrobot 12 is stored in the garage alike structure 28 of the flexible elongated element 20 prior to be removed from the target body part (with or without the flexible elongated element 20).

Thanks to the wire 16, the system 10 according to the present invention enables a safe retrieval of the microrobot 12 in any part of a patient's body, at any distance from the channel 22.

## Claims

1. Delivery and gathering system (10) aimed at delivering or gathering material, energy, information or structural elements from or to at least one target point inside a target body part of a patient, the system comprising:
- a microrobot (12) configured to navigate inside the target body part and aimed at navigating towards the at least one target point,
- a cap (14) aimed at being secured to either a securing element secured to a securing body part of the patient, or directly to the securing body part, the cap (14) comprising a channel (22) which enables to introduce elements or element parts into the target body part, or enables to remove elements or element parts from inside the target body part,
- a wire (16) connected to the microrobot (12) on one end and to the cap (14) on the other end,
- a control unit (18) configured to communicate with the cap (14), the control unit (18) comprising a user interface enabling a user to remotely control and navigate the microrobot (12),
wherein the system further comprise a flexible elongated element (20) configured to cooperate, by sliding, with the wire (16) in order to extend, from the cap (14), through the target body part, towards the target point, the wire (16) being thus aimed at guiding the flexible elongated element (20) towards the target point.

2. System (10) according to the preceding claim, wherein the flexible elongated element (20) displays a general tubular shape, the flexible elongated element (20) being configured to slide around the wire (16).

3. System (10) according to any one of the preceding claims, wherein the flexible elongated element (20) is a catheter configured to cooperate with the wire (16) and the cap (14) in order:
- to deliver material from the cap (14) towards the at least one target point, or
- to gather material from the at least one target point towards the cap (14).

4. System (10) according to the preceding claims, wherein the cap (14) comprises a pump configured to pump fluids and material through the catheter.

5. System (10) according to claim 1, wherein the flexible elongated element (20) is an electrode or a sensor configured to gather information from the at least target point or to deliver energy to said at least target point.

6. System (10) according to any one of the preceding claims, wherein the wire (16) provides energy to the microrobot (12).

7. System (10) according to any one of the preceding claims, wherein the cap (14) comprises a wire winding system in order to control the wire extension inside the target body part, the wire winding system being motorized and controlled by the control unit (18), the wire winding system further comprising at least one monitoring sensor (32).

8. System (10) according to any one of the preceding claims, wherein the cap (14) comprises a flexible elongated element (20) winding system in order to control the extension of the flexible elongated element (20) inside the target body part, the flexible elongated element (20) winding system being motorized and controlled by the control unit (18), the flexible elongated element 20 winding system further comprising at least one monitoring sensor (33).

9. System (10) according to the preceding claim, wherein the flexible elongated element (20) comprises at least one monitoring sensor at its distal end (26).

10. System (10) according to any one of the preceding claims, wherein the flexible elongated element (20) comprises an adaptative diameter on at least one deformable portion (25).

11. System (10) according to the preceding claim, wherein the flexible elongated element (20) comprises a garage alike structure (28) in order to store the micro-robot (12).

12. System (10) according to any one of the preceding claims, wherein the flexible elongated element (20) comprises at least one tracker enabling to monitor, in real time, the extension of the flexible elongated element (20).

13. Material delivery and gathering method implemented by means of the system (10) according to any one of the preceding claims, the method comprising following steps in the order of enunciation:
- securing the cap (14) to the securing body part of the patient,
- programming a microrobot (12) navigation route by means of the user interface of the control unit (18),
- securing, if necessary, one end of the wire (16) to the cap (14),
- securing, if necessary, the other end of the wire (16) to the microrobot (12),
- inserting the microrobot (12) inside the target body part of the patient through the channel (22) of the cap (14),
- inserting the distal end (26) of the flexible elongated element (20) inside the channel (22) of the cap (14),
- setting the flexible elongated element (20) and the wire (16) up for cooperation,
- setting the microrobot into motion towards the at least one target point,
- monitoring the wire (16) extension through the target body part,
- monitoring the flexible elongated element (20) extension through the target body part,
- once the microrobot (12) has reached the at least one target point, activating the material, energy, information or structural elements material delivery or gathering, through the flexible elongated element (20),
- once the delivery or gathering has been completed, either proceed to delivery or gathering to a further target point, or remove the flexible elongated element (20), the wire (16) and the microrobot (12) from the target body part, through the cap (14).

14. Method according to the preceding claim, wherein the microrobot (12) is removed by being pulled through the flexible elongated element (20).

15. Method according to any one of claims 13 or 14, wherein the microrobot (12) is stored in a garage alike structure (28) of the flexible elongated element (20) prior to be removed from the target body part.
